# EUROPEAN PATENT APPLICATION

(11) **EP 2 273 183 A1**
(43) Date of publication of application: **12.01.2011**
(21) Application number: 09742641.5
(22) Date of filing: 09.03.2009
(51) Int. Cl.: F21S 2/00, A61B 1/00, G02B 27/00, G02B 27/18, H04N 5/225, H04N 5/238, H04N 9/04, F21Y 101/02

(54) **ILLUMINATION DEVICE AND ILLUMINATION IMAGING DEVICE**

(30) Priority: 21.04.2008 JP 2008110758
(71) Applicant: Olympus Corporation, Shibuya-ku Tokyo 151-0072 (JP)
(72) Inventor: IMADE, Shinichi, Tokyo 151-0072 (JP)
(74) Representative: Gunzelmann, Rainer
(86) International application number: PCT/JP2009/054388
(87) International publication number: WO 2009/136516

(57) **Abstract**

To provide an illuminating apparatus that can control an illumination period in accordance with an image capturing exposure period without disturbing the color balance, and that can radiate bright illumination light possessing high directivity. The illuminating apparatus (3) includes: a light source (9) including a plurality of light emitting elements (13) aligned in a ring-like manner and emitting light in a radially inward direction; a light guide member (10) having an entering end faces to the radially inward direction of the light source (9) and an emitting end arranged on the center axis of the ring, and guiding light caused to enter the light guide member (10) from the entering end to the emitting end; and a rotary part rotating the light guide member (10) about the center axis of the ring. The light source is structured with a plurality of unit light source blocks P aligned in the circumferential direction. The unit light source blocks P are made up of a plurality of types of the light emitting elements (13) respectively producing light of different colors aligned in the circumferential direction.

## Description

### Technical Field

The present invention relates to an illuminating apparatus and an illuminating/image capturing apparatus.

### Background Art

Conventionally, as an illuminating apparatus of a display apparatus such as a large-screen liquid crystal monitor or a projector, an illuminating apparatus of a scheme in which a multitude of LED light sources aligned in a ring-like manner are lit up successively in a pulsed manner by a large current, and the emitted light is taken in by a light guide part rotating at a high speed so as to be emitted from the rotation center is known (for example, see Patent Citation 1).
In this illuminating apparatus, the produced light is allowed to transmit through or to be reflected from a display apparatus such as of liquid crystal or a DMD (Digital Micromirror Device) to thereby superimpose visual image information, which makes it difficult to switch the visual image information at high speeds. For this reason, the LED light sources of the same color are arranged together so as to be adjacent to each other, and visual images of R (red color), G (green color), and B (blue color) are successively projected.

Patent Citation 1: Japanese Unexamined Patent Application, Publication No. 2004-199024

### Disclosure of Invention

However, when this illuminating apparatus is used as an illuminating apparatus for an apparatus that is associated with an image capturing operation, e.g., an endoscope, it is disadvantageous in the following point.
That is, when the apparatus is associated with the image capturing operation, the light adjustment is required, and both the light shield control and the light amount control are generally exerted with the maximized image capturing exposure period. Accordingly, in a case of taking where a small amount of illumination light suffices, the S/N ratio becomes small and noises become obvious, whereby the quality of the image is degraded.

Further, as to the electronic shutter scheme in which the exposure amount is adjusted on the image capturing apparatus side, it is capable of improving the S/N ratio to thereby obtain a high quality image. However, when it is adopted in the above-described illuminating apparatus, it is disadvantageous in that if the lighting control is exerted in accordance with the electronic shutter of the image capturing apparatus, the color of the illuminated light is biased to any of the colors, and the color balance is impaired.

The present invention has been made in consideration of the circumstances described above, and an object thereof is to provide an illuminating apparatus that can control an illumination period in accordance with an image capturing exposure period without disturbing the color balance, and that can radiate bright illumination light possessing high directivity, and to provide an illuminating/image capturing apparatus that can obtain a high quality image using such an illuminating apparatus without disturbing the color balance.

In order to achieve the object stated above, the present invention provides the following measures.
The first aspect of the present invention is:
an illuminating apparatus, including: a light source that includes a plurality of light emitting elements being aligned in a ring-like manner and emitting light in a radially inward direction; a light guide member that has an entering end facing to the radially inward direction of the light source and an emitting end arranged on a center axis of the ring, and that guides light caused to enter the light guide member from the entering end to the emitting end; and a rotary part that rotates the light guide member about the center axis of the ring, wherein the light source is structured with a plurality of unit light source blocks aligned in a circumferential direction, the unit light source blocks each being made up of a plurality of types of the light emitting elements respectively producing light of different colors aligned in the circumferential direction.

According to the above-described aspect, the light produced by each light emitting element is caused to enter inside the light guide member from the entering end facing to the radially inward direction of the light emitting element, and guided inside the light guide member so as to be emitted from the emitting end on the center axis. By rotating the light guide member by the actuation of the rotary part, each light emitting element that produces the light to be entered inside the light guide member is successively switched. By lighting up each light emitting element in a pulsed manner with a large current at the timing synchronized with the switching, brighter illumination can be achieved as compared with a case where the driving operation is carried out with a rated current, without shortening the life of the light emitting elements.

In this case, the light source is structured with a plurality of unit light source blocks aligned in a circumferential direction, the unit light source blocks each being made up of a plurality of types of the light emitting elements respectively producing light of different colors aligned in the circumferential direction. Therefore, by exerting the lighting control on the unit light source block basis, the light emission amount can be adjusted without disturbing the color balance.

In the above-described aspect, preferably, the plurality of types of the light emitting elements structuring every one of the unit light source blocks are aligned in the circumferential direction in an identical alignment order.
In this manner, the light emission amount can be adjusted without disturbing the color balance, irrespective of whichever light emitting element the configuration of each unit light source block is started from. As a result, the exposure adjustment can be carried out in a further flexible manner.

The second aspect of the present invention is: an illuminating/image capturing apparatus, including: one of the above-described illuminating apparatuses; an image capturing element that takes return light, from a subject, of light produced from the illuminating apparatus; and a control part that controls an exposure period of the image capturing element on a basis of the unit light source block of the illuminating apparatus.
According to the above-described aspect, the controlled exposure period of the image capturing element prevents the exposure while the light emitting element is extinct. This makes it possible to improve the S/N ratio, and to acquire a high quality image. In this case, by the control part controlling the exposure period of the image capturing elements on a unit light source block basis, the exposure can be adjusted without disturbing the color balance.

In the above-described aspect, preferably, a taking cycle of the image capturing element and a rotation cycle of the light guide member caused by the rotary part are different from each other.
In this manner, it becomes possible to prevent an occurrence of the problem of an uneven lighting frequency among the light emitting elements when the light emission amount is adjusted, which would otherwise invite degradation of any specific light emitting element at an earlier stage.

The illuminating apparatus of the present invention exhibits the effect that the illumination period can be controlled in accordance with the image capturing exposure period without disturbing the color balance, and bright illumination light possessing high directivity can be radiated. The illuminating/image capturing apparatus of the present invention exhibits the effect that a high quality image can be obtained without disturbing the color balance using such an illuminating apparatus.

### Brief Description of Drawings

[Fig. 1] An overall structure diagram showing an illuminating/image capturing apparatus according to an embodiment of the present invention.
[Fig. 2] A front view showing, in a partially omitted manner, an illuminating apparatus of the present embodiment installed in the illuminating/image capturing apparatus shown in Fig. 1.
[Fig. 3] A cross-sectional diagram taken along line X-X of the illuminating apparatus shown in Fig. 2.
[Fig. 4] An illustration showing a light source unit structuring the illuminating apparatus shown in Fig. 2.
[Fig. 5A] An illustration for describing a lighting operation of each light source unit in the illuminating apparatus shown in Fig. 2, in which the positional relationship of the adjacent light source units is illustrated.
[Fig. 5B] An illustration for describing the lighting operation of each light source unit in the illuminating apparatus shown in Fig. 2, in which the use efficiency of light emitted from each light source unit is illustrated.
[Fig. 6] A timing chart for describing the operation of the illuminating/image capturing apparatus shown in Fig. 1.
[Fig. 7] A graph showing the relationship between the average output of the image capturing elements of the illuminating/image capturing apparatus shown in Fig. 1 and the image capturing exposure period.
[Fig. 8] A front view showing, in a partially omitted manner, a modification of the illuminating apparatus shown in Fig. 2.
[Fig. 9] A timing chart for describing the operation with a modification of the illuminating/image capturing apparatus shown in Fig. 1.
[Fig. 10] A timing chart showing another illumination pattern by the illuminating apparatus shown in Fig. 2.
[Fig. 11] An illustration showing a modification of the light source unit shown in Fig. 4.

### Explanation of Reference:

- A: opposing inner wall (subject)
- P: unit light source block
- 1: illuminating/image capturing apparatus
- 3: illuminating apparatus
- 4b: image capturing element
- 5: control part
- 9: light source
- 10: light guide member
- 11: rotary part
- 13: light emitting element
- 16a: entering end
- 17a: emitting end

### Best Mode for Carrying Out the Invention

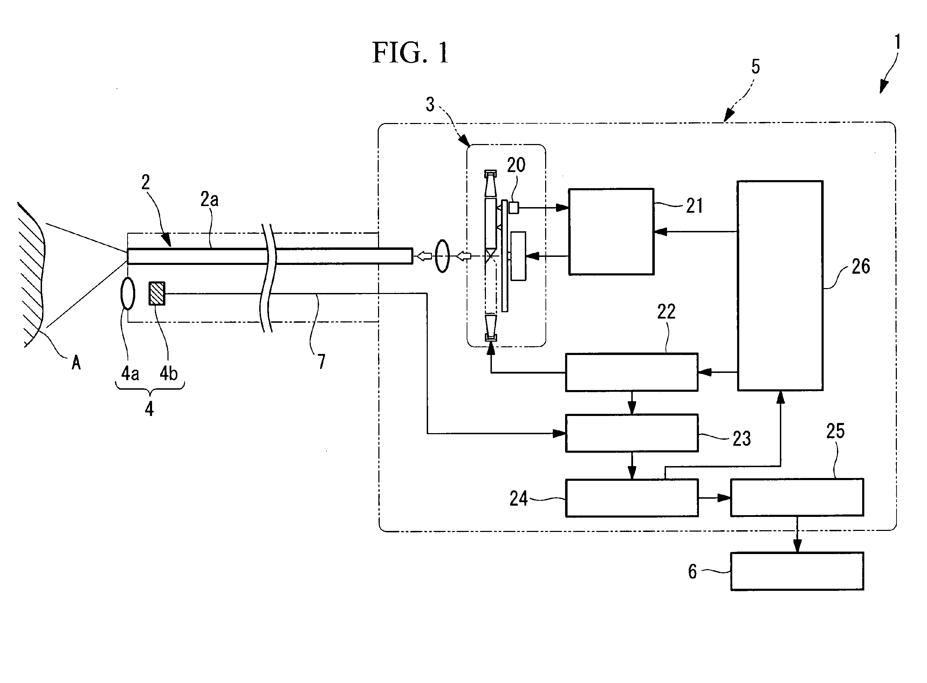

In the following, a description will be given of an illuminating apparatus and an illuminating/image capturing apparatus according to an embodiment of the present invention with reference to Figs. 1 to 7.
An illuminating/image capturing apparatus 1 according to the present embodiment is, e.g., an endoscope apparatus, and includes, as shown in Fig. 1, an insertion part 2 inserted into a body cavity, an illuminating apparatus 3 that produces illumination light to be emitted from the tip of the insertion part 2 toward a body cavity inner wall A, an image capturing apparatus 4 that takes return light such as reflected light, fluorescence or the like at the body cavity inner wall A, a control apparatus 5 that controls above-described components, and a display monitor 6 that displays an acquired visual image.

Across the entire longitudinal directional length of the insertion part 2, a light guide 2a that guides illumination light from the illuminating apparatus 3 is provided. An image capturing apparatus 4 is arranged at the tip part of the insertion part 2.
The image capturing apparatus 4 is provided with an image capturing lens 4a that condenses return light from the body cavity inner wall A, and an image capturing element 4b such as a CCD, which takes the return light condensed by the image capturing lens 4a. The video signal acquired by the image capturing element 4b is transmitted inside the insertion part 2 from the tip side toward the base end side through a wiring 7, so as to be sent to the control apparatus 5.

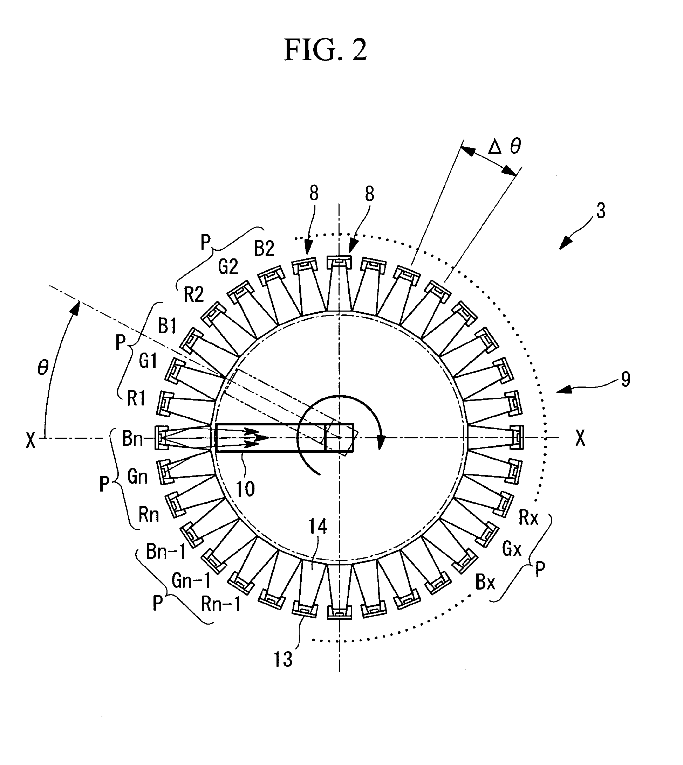

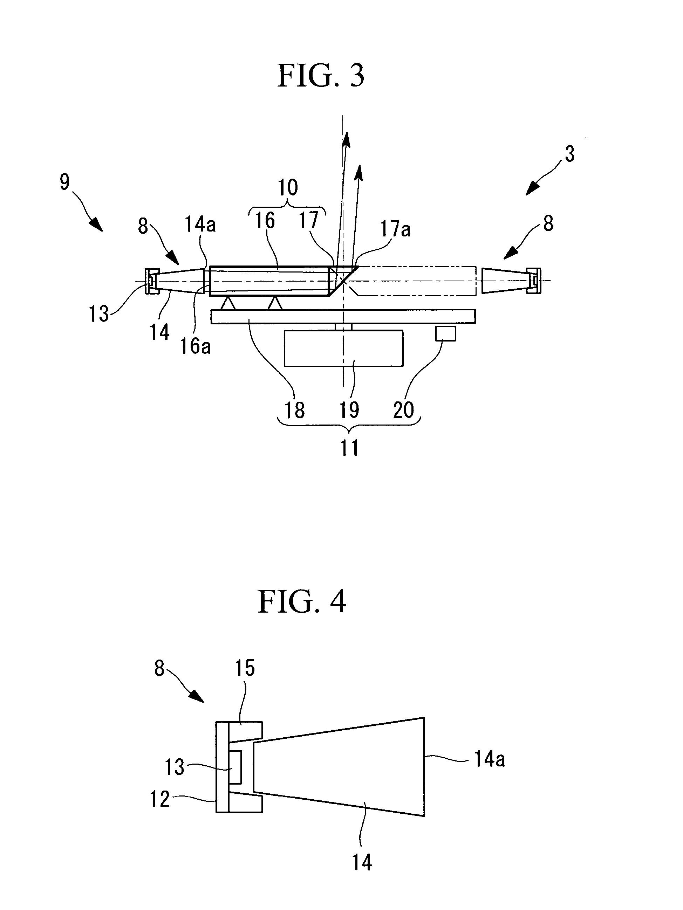

As shown in Figs. 2 and 3, the illuminating apparatus 3 according to the present embodiment includes a light source 9 that is formed to be ring-like by a plurality of light source units 8 aligned in the circumferential direction, and that emits illumination light in the radially inward direction, a light guide member 10 that is arranged on the radially inward side of the ring-like light source 9 so as to extend in the radial direction toward the center of the light source 9, and a rotary part 11 that rotates the light guide member 10 about the center axis of the light source. It is noted that, the position of the light guide member 10 at which its entering end 16a faces to a B-light emitting element 13 (Bn) is defined as the reference position, and an angle of rotation of the light guide member 10 relative to the reference position is represented by θ.

As shown in Fig. 4, each light source unit 8 includes a substrate 12, a light emitting element 13 such as an LED, which is fixed to the substrate 12 and produces illumination light, a taper rod 14 that guides the illumination light produced at the light emitting element 13 while enhancing its directivity, and a reflective member 15 that reflects the illumination light emitted in the peripheral direction from the light emitting element 13 such that the illumination light enters inside the taper rod 14. Each of the light source units 8 is configured to have its taper rod 14 arranged in the radial direction and to have an emitting end 14a of the taper rod 14 arranged in the radially inward direction, so as to be capable of causing the illumination light produced at the light emitting element 13 to emit in the radially inward direction.

As shown in Fig. 3, the light guide member 10 is structured with a parallel rod 16 provided at its one end with the entering end 16a whose area is substantially as great as that of the emitting end 14a of the taper rod 14, and a triangular prism 17 joined to the other end of the parallel rod 16. The triangular prism 17 is arranged on the center axis of the ring-like light source 9, having its emitting end 17a arranged in the direction along the center axis.

Thus, the light guide member 10 is configured, while having its entering end 16a arranged faces to the radially inward direction of the emitting end 14a of the taper rod 14, to cause the illumination light having emitted from the emitting end 14a of the taper rod 14 to enter the entering end 16a to guide the same in the radially inward direction, and to deflect the light guiding direction by 90° through the triangular prism 17, so as to cause the illumination light to emit along the center axis of the light source 9.
The rotary part 11 includes a disk-like support member 18 that supports the light guide member 10, a motor 19 that rotates the support member 18 about the center axis, and a rotation position sensor 20 that detects the rotation position of the light guide member 10.

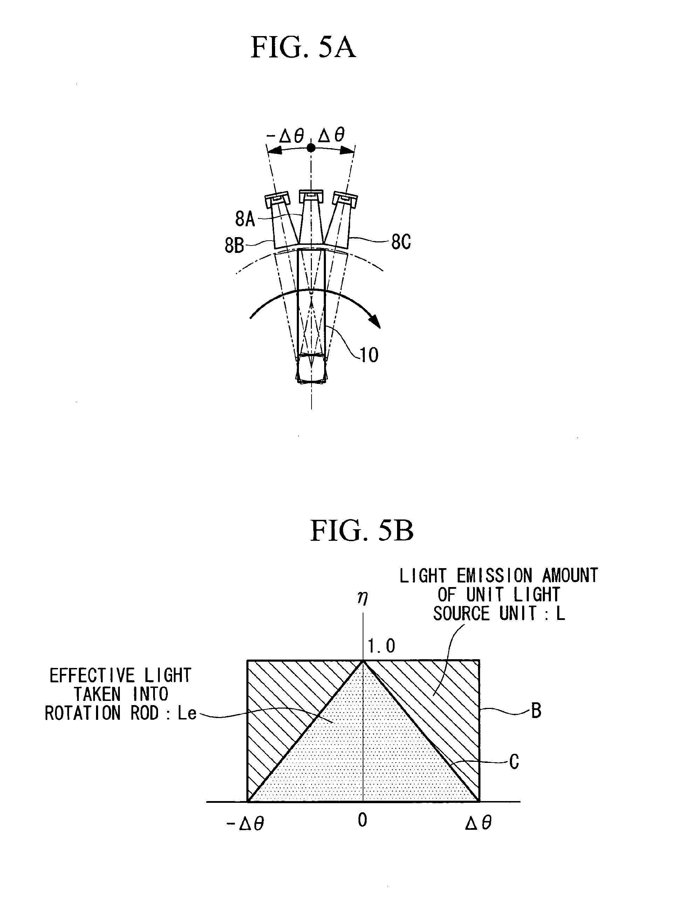

More specifically, as shown in Fig. 5A, the description is given by defining the relative angle formed between adjacent light source units 8 as Δ θ, and within a range of ± Δ θ with reference to the central specific light source unit 8A. As denoted by Reference B in Fig. 5B, the specific light source unit 8A is configured to be lit within a range of ± Δ θ in a pulsed manner. In this case, the light amount of the illumination light entering the light guide member 10 from the specific light source unit 8A varies as represented by a triangle region C in Fig. 5B.

That is, the light amount gradually increases, starting from the state where the entering end 16a of the light guide member 10 faces to the front face of the emitting end 14a of the taper rod 14 of the adjacent light source unit 8B, in accordance with the rotation of the light guide member 10. The light amount reaches its maximum at the position where the entering end 16a of the light guide member 10 faces to the front face of the emitting end 14a of the taper rod 14 of the light source unit 8A. Thereafter, the light amount gradually decreases in accordance with the rotation of the light guide member 10. This holds true for every light source unit 8. Accordingly, as to the illumination light of the light source units 8, the following is established: the use efficiency η = Le/L.

As to the illuminating apparatus 3 according to the present embodiment, a plurality of light source units 8 respectively having a plurality of types of light emitting elements 13 respectively producing light of different colors are aligned in the circumferential direction, to constitute a unit light source block P. The illuminating apparatus 3 is made up of a plurality of such unit light source blocks P aligned in the circumferential direction.
For example, in the exemplary case shown in Fig. 2, the unit light source block P is structured with three light source units 8 respectively having the light emitting elements 13 (Rx (x = 1, 2, ..., n), Gx, Bx) that respectively produce illumination light of R (red color), G (green color), and B (blue color). A plurality of sets of such unit light source blocks P are aligned in the circumferential direction. The light emitting elements 13 in every unit light source block P are aligned in order of R, G, and B, clockwise.

As shown in Fig. 1, the control apparatus 5 includes a rotary drive control part 21 that controls the rotation of the light guide member 10 in the illuminating apparatus 3, a light source control part 22 that controls a light emission timing of the light emitting elements 13 in the illuminating apparatus 3, an image capturing element control part 23 that controls an exposure period of the image capturing element 4b in accordance with the light emission timing set by the light source control part 22, a video signal processing part 24 that processes a video signal acquired by the image capturing element 4b, a display signal processing part 25 that processes for a display purpose the visual image processed by the video signal processing part 24, and a system control part 26 that calculates an exposure amount in accordance with the brightness of the image extracted by the video signal processing part 24 to output a command signal to the rotary drive control part 21 and the light source control part 22.

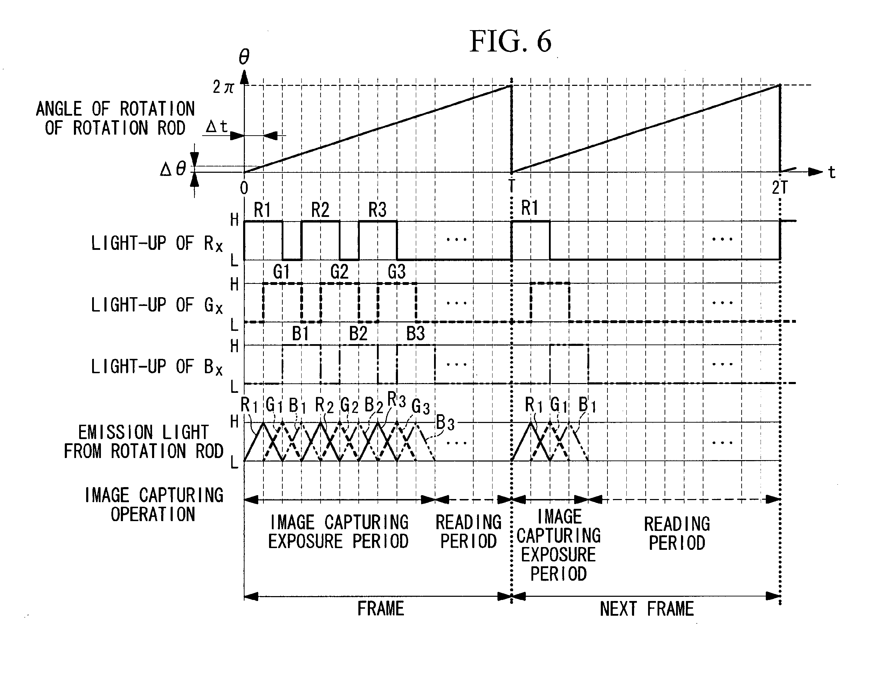

When the system control part 26 determines that the visual image sent from the video signal processing part 24 is dark and the exposure amount is insufficient, the system control part 26 commands the light source control part 22 to increase the number of lit up unit light source block(s) P. On the other hand, when the system control part 26 determines that the visual image sent from the video signal processing part 24 is bright and the exposure amount is excessively great, the system control part 26 commands the light source control part 22 to decrease the number of lit up unit light source blocks P. In the exemplary case shown in the left half part in Fig. 6, three unit light source blocks P are lit up, whereas in the exemplary case shown in the right half part, one unit light source block P is lit up.

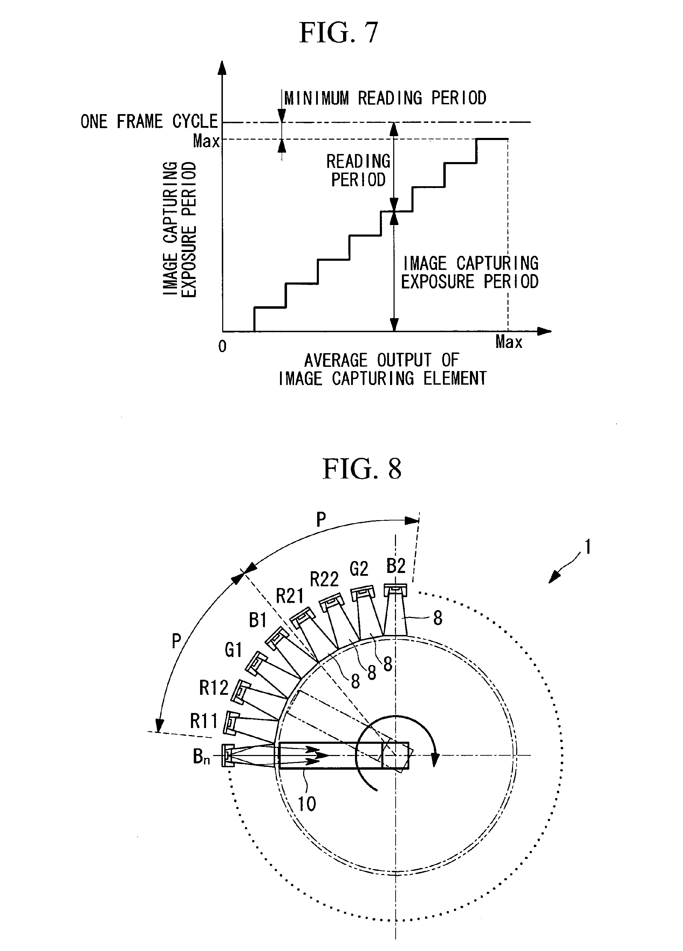

The image capturing element control part 23 receives the light emission timing set by the light source control part 22, and, sets a period from the start of light-up of the three unit light source blocks P until extinction thereof in the case of the left side part in Fig. 6, and a period from the start of light-up of one unit light source block P until extinction thereof in the case of the right side part, as the exposure period executed by the image capturing element 4b. It is noted that the remainder of the period during one frame is set as a data reading period from the image capturing element 4b. As a result, the output average of the image capturing element 4b and the exposure period vary stepwise in accordance with the relationship shown in Fig. 7.

In the following, a description will be given of the operation of the illuminating/image capturing apparatus 1 according to the present embodiment structured in this manner.
In order to carry out a taking operation using the illuminating/image capturing apparatus 1 according to the present embodiment, the insertion part 2 is inserted inside a body cavity, such that the tip of the insertion part 2 opposes to the body cavity inner wall A near the affected area to be observed.

In this state, based on a command signal from the system control part 26, the rotary drive control part 21 actuates the motor 19 to rotate the light guide member 10, and the light source control part 22 lights up the light emitting elements 13 in a pulsed manner in accordance with the commanded light emission timing.
The illumination light produced from the light emitting elements 13 is caused to enter inside the light guide member 10 from the entering end 16a faces to the radially inward direction of the light emitting element 13, to be guided inside the light guide member 10, and to be emitted from the emitting end 17a on the center axis. By rotating the light guide member 10 by the actuation of the motor 19, each light emitting element 13 that produces the illumination light to be entered inside the light guide member 10 is successively switched. By lighting up each light emitting element 13 in a pulsed manner with a large current at the timing synchronized with the switching, brighter illumination can be achieved as compared with a case where the driving operation is carried out with a rated current, without shortening the life of the light emitting elements 13.

In the present embodiment, the light source control part 22 exerts lighting control over the light emitting elements 13 on a unit light source block P basis. For example, in a case where the light amount initially set is the minimum light amount, the light source control part 22 lights up only a single unit light source block P. That is, while the light guide member 10 rotates one revolution, one each of the R-, G-, and B-light emitting elements 13 are lit up and the other light emitting elements 13 are extinct, whereby illumination light of the minimum light amount is produced.

Then, the image capturing element control part 23 receives the light emission timing set by the light source control part 22, and sets the period during which the three light emitting elements 13 constituting a single unit light source block P are lit up as the exposure period, and a visual image is acquired by the image capturing element 4b. Then, the acquired video signal is transmitted to the system control part 26 to have its exposure amount determined. When the exposure amount is insufficient, the number of lit up unit light source block(s) P is increased; when the exposure amount is excessively great, the number of lit up unit light source blocks P is decreased.

In this case, with the illuminating/image capturing apparatus 1 according to the present embodiment, the lighting operation of the light emitting elements 13 in the illuminating apparatus 3 is controlled on a unit light source block P basis, which is constituted of three adjacent R-, G-, and B-light emitting elements 13. Therefore, without disturbing the color balance of the radiated illumination light, the light emission amount thereof can be adjusted.
In the present embodiment, because the alignment order of the light emitting elements 13 in every unit light source block P is identical, from whichever R-, G-, or B- light emitting element 13 the lighting operation is started, the unit light source block P can be structured from that starting position so that the lighting control can be exerted on a unit light source block P basis. This makes it possible to more finely adjust the exposure period of the image capturing element 4b.

With the illuminating/image capturing apparatus 1 according to the present embodiment, the exposure period of the image capturing element 4b is adjusted in synchronization with the light emission timing of the light emitting elements 13 set by the illuminating apparatus 3. Therefore, the present invention is advantageous in that it is capable of suppressing an increase in noises caused by the exposure of the image capturing element 4b in a state where the light emitting elements 13 are not emitting light, whereby a visual image of a high S/N ratio and of a high quality can be obtained.

It is noted that, in the illuminating/image capturing apparatus 1 according to the present embodiment, it has been described that the three R-, G-, and B-light emitting elements 13 in every unit light source block P are aligned in the identical order. Alternatively, they may be aligned in a different order. In such a case also, by exerting the lighting control of the light emitting elements on a unit light source block P basis, the light amount adjustment can be carried out without disturbing the color balance:

It has been described that the unit light source block P is structured with three light source units 8 respectively having three types, i.e., R-, G-, and B-, light emitting elements 13. Alternatively, as shown in Fig. 8, the color structure may be altered. In the exemplary case shown in Fig. 8, the unit light source block P is structured with four light source units 8 respectively having two R-, one G-, and one B-light emitting elements. The present invention is not limited thereto, and any unit light source block P having an arbitrary color structure may be employed.
In this case also, so long as the alignment order of the light emitting elements 13 in every unit light source block P is identical, from whichever light emitting element 13 the lighting is started, the unit light source block P can be structured from that starting position, to exert the lighting control on a unit light source block P basis.

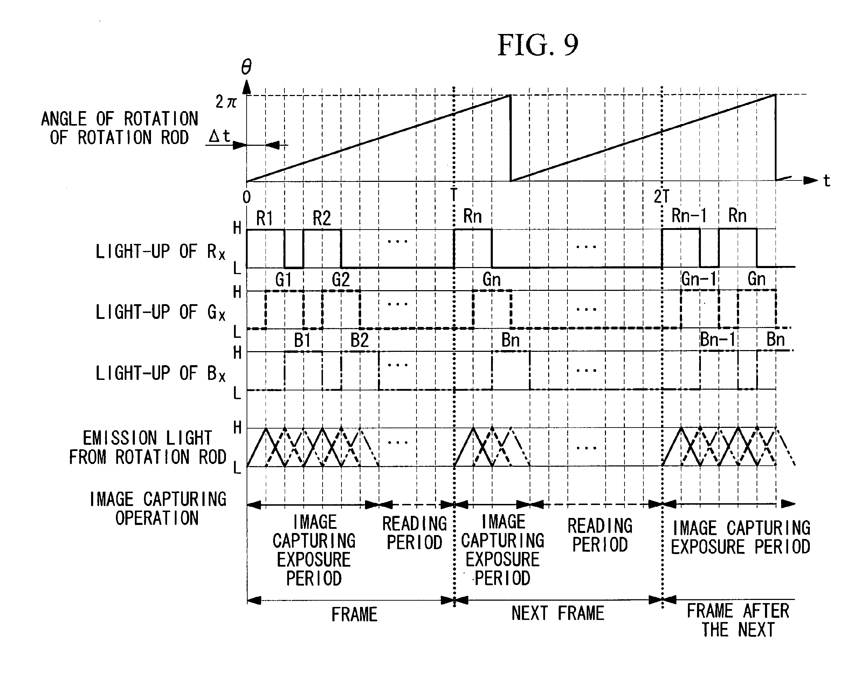

In the present embodiment, the exemplary case in which one revolution of the light guide member 10 caused by the motor 19 and one frame (image capturing cycle) of a visual image are matched with each other has been shown. However, as shown in Fig. 9, the rotation cycle of the light guide member 10 and the image capturing cycle may be different from each other. In the exemplary case shown in Fig. 9, the rotation cycle of the light guide member 10 is set to be slightly longer than the image capturing cycle.

Thus, for example, adjustment of the light emission amount achieved by adjusting the number of the light emitting element(s) 13 to be lit since the start of the image capturing cycle can similarly prevent the occurrence of the problem of uneven lighting frequency among the light emitting elements 13. As a result, it becomes possible to light up every light emitting element 13 evenly, so as not to shorten the life of any specific light emitting element 13 only.

By using a similar technique, configuring such that every light emitting element 13 produces illumination light of one single color (for example, white), and causing the image capturing cycle and the rotation cycle to be asynchronous, it becomes possible to prevent the life of any specific light emitting element 13 from being shortened.

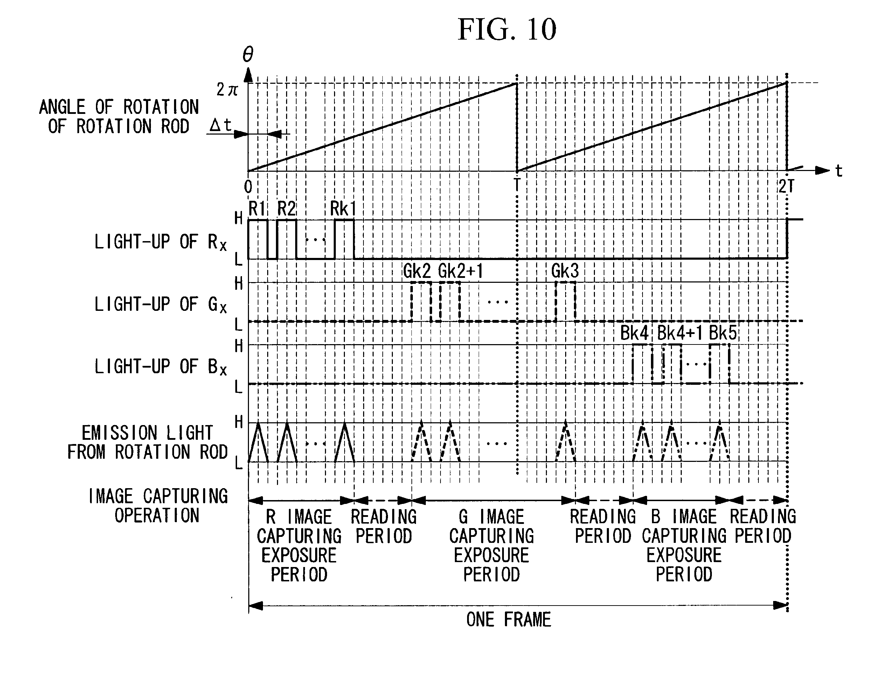

As to the illuminating apparatus 3 and the illuminating/image capturing apparatus 1 according to the present embodiment, the description has been given of the case where the light emitting elements 13 are lit up in order of the alignment order of the light source units 8. Alternatively, as shown in Fig. 10, the light emitting elements 13 of three types, i.e., R-, G-, and B-, light emitting elements 13, may be lit up together on an illumination light color basis. In the exemplary case shown in Fig. 10, only the R-light emitting elements 13 (Rx) are lit up first, then only the G-light emitting elements 13 (Gx) are lit up, and finally only the B-light emitting elements 13 (Bx) are lit up. In this case, by adjusting the number of the lit up light emitting elements 13 of respective colors, the exposure period for each color can be changed.
The illumination method can be freely selected in consideration of the light emission amount of the light emitting elements 13 of each type and the image capturing sensitivity of the image capturing element 4b, such that a desired video signal can be shot.

In the exemplary case shown in Fig. 10, the image capturing period for one frame is set so as to be allotted to two revolutions of the light guide member 10. However, the present invention is not limited thereto, and it can be set arbitrarily.
It is noted that, in connection with the endoscope apparatus, in some cases, the insertion part (scope) 2 is replaced in accordance with the site to be observed. In such a case, in a situation where change of the taking condition is necessitated depending on the image capturing element 4b provided to the insertion part 2 and the like, the image capturing period and the reading period can easily be changed.

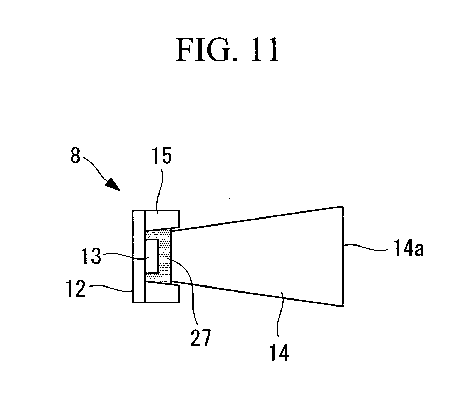

In the present embodiment, the description has been given of the case where each light source unit 8 has the light emitting elements 13 that produce illumination light of specific colors. Alternatively, as shown in Fig. 11, as each light emitting element 13, it is also possible to employ a combination of an element that produces excitation light of an identical wavelength band (for example, ultraviolet light) and a fluorescent substance 27 that is excited by the excitation light and produces fluorescence. In this case, by allotting a different fluorescent substance 27 for each light source unit 8, it becomes possible to produce illumination light of different colors.

## Claims

1. An illuminating apparatus, comprising:
a light source that includes a plurality of light emitting elements being aligned in a ring-like manner and emitting light in a radially inward direction;
a light guide member that has an entering end faces to the radially inward direction of the light source and an emitting end arranged on a center axis of the ring, and that guides light caused to enter the light guide member from the entering end to the emitting end; and
a rotary part that rotates the light guide member about the center axis of the ring, wherein
the light source is structured with a plurality of unit light source blocks aligned in a circumferential direction, the unit light source blocks each being made up of a plurality of types of the light emitting elements respectively producing light of different colors aligned in the circumferential direction.

2. The illuminating apparatus according to claim 1, wherein
the plurality of types of the light emitting elements structuring every one of the unit light source blocks are aligned in the circumferential direction in an identical alignment order.

3. An illuminating/image capturing apparatus, comprising:
the illuminating apparatus according to one of claim 1 and claim 2;
an image capturing element that takes return light, from a subject, of light produced from the illuminating apparatus; and
a control part that controls an exposure period of the image capturing element on a basis of the unit light source block of the illuminating apparatus.

4. The illuminating/image capturing apparatus according to claim 3, wherein
a taking cycle of the image capturing element and a rotation cycle of the light guide member caused by the rotary part are different from each other.
